# EUROPÄISCHE PATENTSCHRIFT

(11) **EP 0 640 593 B1**
(45) Veröffentlichungstag und Bekanntmachung des Hinweises auf die Patenterteilung: **29.12.1997**
(21) Anmeldenummer: 94111575.0
(22) Anmeldetag: 25.07.1994
(51) Int. Cl.: C07D 233/54, C07D 213/30, C07C 279/22, A61K 31/41, A61K 31/44

(54) **Substituierte Benzoylguanidine, Verfahren zu ihrer Herstellung, ihre Verwendung als Medikament oder Diagnostikum sowie sie enthaltendes Medikament**
Substituted benzoylguanidines, process for their preparation, their use as medicine or in diagnostic and medicines containing them
Benzoylguanidines substituées, procédé pour leur préparation, leur utilisation comme médicament ou en diagnostic et médicaments les contenant

(30) Priorität: 31.07.1993 DE 4325822
(43) Veröffentlichungstag der Anmeldung: 01.03.1995
(73) Patentinhaber: HOECHST AKTIENGESELLSCHAFT, 65929 Frankfurt am Main (DE)
(72) Erfinder: Kleemann, Heinz-Werner, Dr., D-61350 Bad Homburg (DE); Lang, Hans-Jochen, Dr., D-65719 Hofheim/Taunus (DE); Schwark, Jan-Robert, Dr., D-65929 Frankfurt (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 416 499
- EP-A- 0 556 672
- EP-A- 0 556 674
- EP-A- 0 577 024
- EP-A- 0 602 522
- EP-A- 0 602 523
- EP-A- 0 603 650
- WO-A-93/04048
- FR-M- 5 704

## Beschreibung

Substituierte Benzoylguanidine, Verfahren zu ihrer Herstellung, ihre Verwendung als Medikament oder Diagnostikum sowie sie enthaltendes Medikament

Die Erfindung betrifft Benzoylguanidine der Formel I 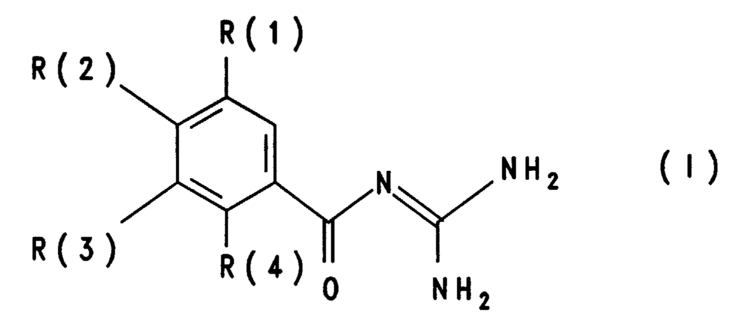 worin bedeuten:
- R(1): Wasserstoff, F, Cl, Br, l, -NO₂, -C≡N, -Xₒ-(CH₂)ₚ-(CF₂)_{q}-CF₃, R(5)-SOₘ-, R(6)-CO-, R(6)R(7)N-CO- oder R(6)R(7)N-SO₂-;
X Sauerstoff, -S- oder NR(14);
m Null, 1 oder 2;
o Null oder 1;
p Null, 1 oder 2;
q Null, 1, 2, 3, 4, 5 oder 6;
R(5) und R(6) (C₁-C₈)-Alkyl, (C₃-C₆)-Alkenyl, -CₙH₂ₙ-R(8) oder CF₃;
n Null, 1, 2, 3 oder 4;
R(8) (C₃-C₇)-Cycloalkyl, Phenyl, welches nicht substituiert ist oder substituiert mit 1 bis 3 Substituenten ausgewählt aus der Gruppe bestehend aus F, Cl, CF₃, Methyl, Methoxy und NR(9)R(10);
R(9) und R(10)
H oder (C₁-C₄)-Alkyl; oder
R(6) Wasserstoff;
R(7) Wasserstoff oder (C₁-C₄)-Alkyl;
oder
R(6) und R(7) gemeinsam 4 oder 5 Methylengruppen sein können, von denen eine CH₂-Gruppe durch Sauerstoff, S, NH, N-CH₃ oder N-Benzyl ersetzt sein kann;
- R(2): 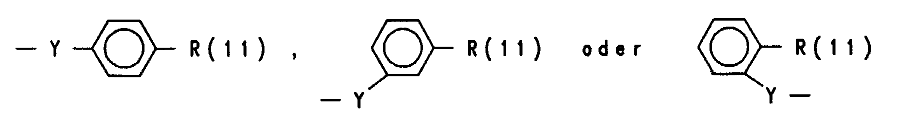 R(11) (C₁-C₉)-Heteroaryl, das über C oder N verknüpft ist und das unsubstituiert oder substituiert ist mit 1 bis 3 Substituenten ausgewählt aus der Gruppe bestehend aus F, Cl, CF₃, CH₃, Methoxy, Hydroxy, Amino, Methylamino, Dimethylamino und Benzyl;
Y Sauerstoff, -S- oder NR(12);
R(12) H oder (C₁-C₄)-Alkyl;
- R(3): wie R(1) definiert;
oder
- R(3): (C₁-C₆)-Alkyl, -X-R(13);
X Sauerstoff, -S- oder NR(14);
R(14) H oder (C₁-C₃)-Alkyl;
R(13) H, (C₁-C₆)-Alkyl, (C₃-C₈)-Cycloalkyl, -C_{b}H_{2b}-R(15) mit b gleich Null, 1, 2, 3 oder 4;
oder
R(13) und R(14) auch gemeinsam 4 oder 5 Methylengruppen, von denen eine CH₂-Gruppe durch Sauerstoff, S, NH, N-CH₃ oder N-Benzyl ersetzt sein kann;
R(15) Phenyl, das unsubstituiert oder substituiert ist mit 1 - 3 Substituenten ausgewählt aus der Gruppe bestehend aus F, Cl, CF₃, Methyl, Methoxy und NR(9)R(10);
R(9) und R(10)
H oder (C₁-C₄)-Alkyl;
- R(4): Wasserstoff, -OR(16), -NR(16)R(17) oder CᵣF₂ᵣ₊₁;
R(16), R(17) unabhängig Wasserstoff oder (C₁-C₃)-Alkyl;
r 1, 2, 3 oder 4;
sowie deren pharmazeutisch verträgliche Salze.

Bevorzugt sind Verbindungen der Formel I, in der bedeuten:
- R(1): Wasserstoff, F, Cl, -C≡N, -CF₃, R(5)-SOₘ-, R(6)-CO-, R(6)R(7)N-CO- oder R(6)R(7)N-SO₂-;
m Null, 1 oder 2;
R(5) und R(6) (C₁-C₈)-Alkyl, (C₃-C₄)-Alkenyl, -CₙH₂ₙ-R(8), -CF₃;
n Null oder 1;
R(8) (C₃-C₆)-Cycloalkyl oder Phenyl, welches unsubstituiert ist oder substituiert mit 1 - 3 Substituenten aus der Gruppe bestehend aus F, Cl, CF₃, Methyl, Methoxy und NR(9)R(10);
R(9) und R(10)
H oder Methyl;
oder
R(6) auch Wasserstoff;
R(7) Wasserstoff oder Methyl;
- R(3): Wasserstoff, Methyl, Cyano, -CF₃, F, Cl;
und die übrigen Reste wie oben definiert sind,
sowie deren pharmazeutisch verträgliche Salze.

Besonders bevorzugt sind Verbindungen I, in denen bedeuten:
- R(1): Wasserstoff, F, Cl, -C≡N, -CF₃, R(5)-SOₘ-, R(6)-CO-, R(6)R(7)N-CO- oder R(6)R(7)N-SO₂-;
m Null, 1 oder 2;
R(5) Methyl oder CF₃;
R(6), R(7) unabhängig voneinander Wasserstoff, Methyl oder CF₃;
- R(2): 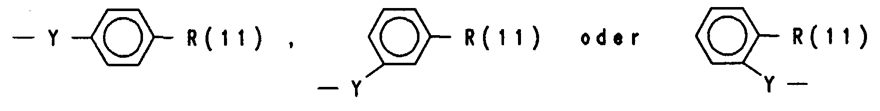 R(11) (C₁-C₉)-Heteroaryl, das über C oder N verknüpft ist und das unsubstituiert oder substituiert ist mit 1 bis 2 Resten ausgewählt aus der Gruppe bestehend aus F, Cl, CF₃, CH₃, Methoxy, Dimethylamino und Benzyl;
Y Sauerstoff, -S- oder NR(12)-;
R(12) Wasserstoff oder Methyl;
- R(3): Methyl, Cyano, Trifluormethyl, F, Cl oder Wasserstoff;
- R(4): Wasserstoff, OH, NH₂ oder CF₃;
sowie deren pharmazeutisch verträgliche Salze.

Insbesondere bevorzugt sind Verbindungen der Formel I, in der bedeuten
- R(1): Wasserstoff, F, Cl, -C≡N, -CF₃, R(5)-SO₂, R(6)-CO-, R(6)R(7)N-CO- oder R(6)R(7)N-SO₂-;
R(5) Methyl oder CF₃;
R(6), R(7) unabhängig voneinander Wasserstoff, Methyl oder CF₃;
- R(2): 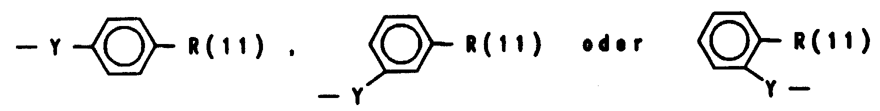 R(11) (C₁-C₅)-Heteroaryl, das über C oder N verknüpft ist und das unsubstituiert oder substituiert ist mit 1 bis 2 Resten ausgewählt aus der Gruppe bestehend aus F, Cl, CF₃, CH₃, Methoxy, Dimethylamino und Benzyl;
Y Sauerstoff, -S- oder NR(12)-;
R(12)
Wasserstoff oder Methyl;
- R(3): Methyl, Cyano, Trifluormethyl, F, Cl oder Wasserstoff;
- R(4): Wasserstoff, OH, NH₂ oder CF₃;
sowie deren pharmazeutisch verträgliche Salze.

Ganz besonders bevorzugt sind Verbindungen der Formel I, in der bedeuten:
- R(1): Wasserstoff, F, Cl, -C≡N, -CF₃, R(5)-SO₂-, R(6)-CO-, R(6)R(7)N-CO- oder A(6)A(7)N-SO₂-;
R(5) Methyl oder CF₃;
R(6), R(7) unabhängig voneinander
H, Methyl oder CF₃;
- R(2): 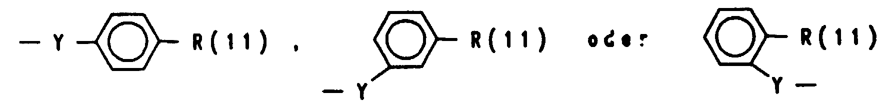 R(11) Pyrrolyl, Imidazolyl, Pyrazolyl, Pyridyl, das unsubstituiert oder substituiert ist mit 1 bis 2 Resten ausgewählt aus der Gruppe bestehend aus F, Cl, CF₃, CH₃, Methoxy, Dimethylamino und Benzyl;
Y Sauerstoff, -S- oder NR(12)-;
R(12) Wasserstoff oder Methyl;
- R(3): Methyl, Cyano, Trifluormethyl, F, Cl oder Wasserstoff;
- R(4): Wasserstoff, OH, NH₂ oder CF₃;
sowie deren pharmazeutisch verträgliche Salze.

Die bezeichneten Alkylreste können sowohl geradkettig wie verzweigt vorliegen.

Unter (C₁-C₉)-Heteroaryl werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Die Erfindung betrifft weiterhin ein Verfahren zur Herstellung der Verbindung I, dadurch gekennzeichnet, daß man Verbindungen der Formel II 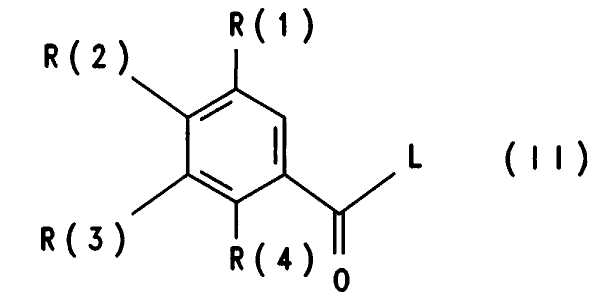 mit Guanidin umsetzt, worin R(1) bis R(4) die angegebene Bedeutung besitzen und L für eine leicht nucleophil substituierbare leaving group steht, und daß man gegebenenfalls in ein pharmakologisch verträgliches Salz überführt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1 -Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 - 367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit
O-[(Cyano(ethoxycarbonyl)methylen)amino]-1,1,3,3-tetramethyluronium-tetrafluorborat ("TOTU")[Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden, indem man beispielsweise 4-(bzw. 5-)Halogen-3-chlor-sulfonylbenzoesäuren mit Ammoniak oder Aminen in 3-Aminosulfonyl-4-(bzw. 5-)halogen-benzoesäuren bzw. mit einem schwachen Reduktionsmittel wie Natriumbisulfit und anschließender Alkylierung in 3-Alkylsulfonyl-4-(bzw. 5-)halogen-benzoesäuren überführt und die erhaltenen Benzoesäuren nach einer der oben beschriebenen Verfahrensvarianten zu erfindungsgemäßen Verbindungen I umgesetzt werden.

Die Einführung einiger Substituenten in 4- und 5-Stellung gelingt durch literaturbekannte Methoden des Palladium-vermittelten cross-couplings von Arylhalogeniden mit beispielsweise Organostannanen, Organoboronsäuren oder Organoboranen oder Organokupfer- bzw. -zink-verbindungen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 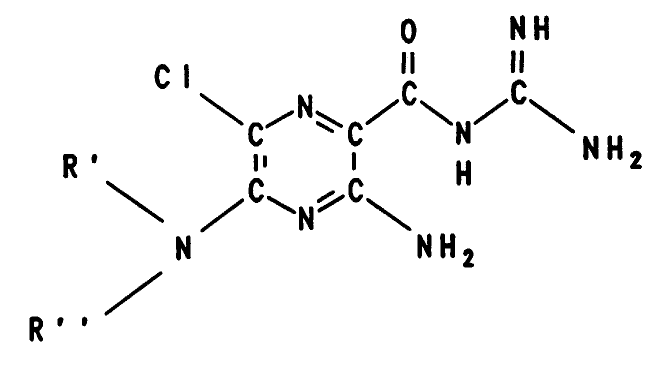
Amilorid: R',R'' = H
Dimethylamilorid: R',R'' = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen (Circulation 79, 1257 bis 1263 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts)). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff-Atom tragen. In der deutschen Patentanmeldung P 42 04 575.4 (HOE 92/F 034) werden 3,5-substituierte Benzoylguanidine vorgeschlagen, in welchen aber der Substituent R(2) nicht die nach der vorliegenden Erfindung beanspruchten Bedeutungen hat.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften gegen solche Arrhythmien aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- NBS: N-Bromsuccinimid
- AIBN: *a,a*-Azo-bis-isobutyronitril
- El: electron impact
- DCI: Desorption-Chemical Ionisation
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent
- ES: Elektrospray-lonisation

### Experimenteller Teil

### Allgemeine Vorschrift zur Herstellung von Benzoyl-guanidinen (I) Variante A: aus Benzoesäuren (II, L = OH)

0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I) Variante B: aus Benzoesäure-alkylestern (II, L = O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotavapor) abdestilliert, in 300 ml EE aufgenommen und 3x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1

### 4-(1-Benzylimidazol-2-yl)phenoxy-3-methylsulfonyl-benzoylguanidin, Dihydrochlorid

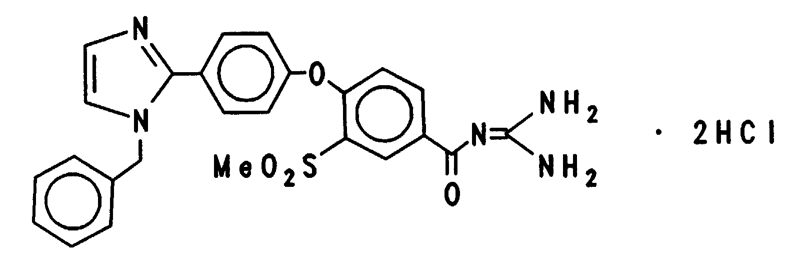

### a) 4-(2-Methoxy-ethoxymethoxy)-benzaldehyd

0,16 mol 4-Hydroxybenzaldehyd und 0,32 mol Ethyldiisopropylamin werden in 250 ml CH₂Cl₂ (wasserfrei) gelöst, und dann werden bei Raumtemperatur 0,24 mol 2-Methoxy-ethoxymethylchlorid zugetropft. 2 Stunden lang wird bei dieser Temperatur gerührt, anschließend das Solvens im Vakuum entfernt. Mit 200 ml EE wird aufgenommen und je 1x mit 200 ml Na₂CO₃-Lösung und 200 ml NaH₂PO₄-Lösung gewaschen, über Na₂SO₄ getrocknet und das Solvens im Vakuum entfernt. Man erhält 34 g eines hellbraunen Öls, das ohne Reinigung weiter umgesetzt wird.
R_{f} (MTB) = 0,53 MS(DCI): 211 (M+1)

### b) 2-[4-(2-Methoxy-ethoxymethoxy)phenyl]-imidazol

83 mmol Aldehyd 1a), 0,46 mol 30 % wäßrige Glyoxal-Lösung und 0,39 mol Natriumacetat werden bei Raumtemperatur in 250 ml gesättigter wäßriger NH₃-Lösung sowie 500 ml MeOH gelöst. 24 Stunden wird bei Raumtemperatur stehen gelassen, anschließend das MeOH im Vakuum entfernt, 200 ml Na₂CO₃-Lösung zugegeben und 3x mit je 300 ml EE extrahiert. Über Na₂SO₄ wird getrocknet und das Solvens im Vakuum entfernt. Chromatographie an Kieselgel mit EE/MeOH 10:1 liefert 13 g eines kristallinen Produkts. mp. 63 bis 64°C
R_{f} (EE/MeOH 10:1) = 0,43 MS (DCl): 249 (M+1)

### c) 1-Benzyl-2-[4-(2-Methoxy-ethoxymethoxy)phenyl]-imidazol

11 mmol Imidazol 1b, werden in 50 ml DMF gelöst und bei Raumtemperatur 11 mmol NaH zugegeben. 30 Minuten wird bei dieser Temperatur nachgerührt, 11 mmol Benzylbromid zugespritzt und weitere 2 Stunden bei Raumtemperatur gerührt. Anschließend wird auf 200 ml NaCl-Lösung gegossen, 3x mit je 100 ml MTB extrahiert und über Na₂SO₄ getrocknet. Das Solvens wird im Vakuum entfernt, und Chromatographie an Kieselgel mit CH₂Cl₂/MeOH 15:1 liefert 3,8 g eines blaßgelben Öls.
R_{f} (CH₂Cl₂/MeOH 15:1) = 0,27 MS (DCl): 339 (M + 1)

### d) 1-Benzyl-2-(4-hydroxyphenyl)-imidazol

10 mmol des Ethers 1e) werden in 200 ml CH₂Cl₂ (wasserfrei) gelöst und bei Raumtemperatur tropfenweise mit 10 mmol TiCl₄ versetzt. 24 Stunden wird bei Raumtemperatur gerührt, anschließend wird NaHCO₃-Lösung zugetropft, bis pH = 7 erreicht ist. Das Titandioxid wird abfiltriert, die Phasen getrennt und die wäßrige Phase noch 3x mit 200 ml EE extrahiert. Die organischen Phasen werden vereinigt, über Na₂SO₄ getrocknet und die Solventien im Vakuum entfernt. Chromatographie an Kieselgel mit EE/MeOH 10:1 liefert 1,1 g eines farblosen Schaums.
R_{f} (EE/MeOH 10: 1) = 0,35 MS (DCI): 251 (M + 1)

### e) 4-(1-Benzylimidazol-2-yl)phenoxy-3-methylsulfonyl-benzoesäuremethylester

3,6 mmol Phenol 1d), 3,6 mmol 4-Fluor-3-methylsulfonylbenzoesäuremethylester und 1,5 g K₂CO₃ werden in 40 ml DMF (wasserfrei) unter Argon 2 Stunden bei 130°C gerührt. Man läßt abkühlen, gießt auf 200 ml H₂O und extrahiert 3x mit je 200 ml EE. Die EE-Phase wird noch mit 100 ml NaHCO₃-Lösung gewaschen, über Na₂SO₄ getrocknet und das Solvens im Vakuum entfernt. Chromatographie an Kieselgel mit EE liefert 1,2 g eines farblosen Öls.
R_{f} (EE) = 0,19 MS (ES): 463 (M + 1)

### f) 4-(1-Benzylimidazol-2-yl)phenoxy-3-methylsulfonyl-benzoylguanidin, Dihydrochlorid

1,5 mmol Ester 1e) und 7,5 mmol Guanidin werden in 10 ml Isopropanol nach Variante B umgesetzt. 400 mg farbloser Schaum.
R_{f} (EE/MeOH 3:1) = 0,22 MS (FAB): 490 (M + 1)

### Beispiel 2

### 4-(Imidazol-2-yl)phenoxy-3-methylsulfonyl-benzoylguanidin, Dihydrochlorid

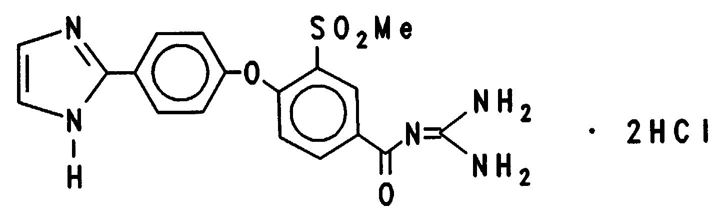

### a) 4-(Imidazol-2-yl)phenoxy-3-methylsulfonyl-benzoesäure-2-hydroxyethylester

1,4 mmol N-Benzylimidazol-Derivat 1e), 14 mmol Ammoniumformiat und 100 mg Pd/C (10 % Pd) werden in 20 ml Ethylenglycol 4 Stunden unter Rückfluß erhitzt. Man läßt abkühlen, gießt in 100 ml Na₂CO₃-Lösung und extrahiert 3x mit je 50 ml EE. Über MgSO₄ wird getrocknet und das Solvens im Vakuum entfernt. Chromatographie an Kieselgel mit EE/MeOH 8:1 liefert 400 mg eines farblosen Feststoffs.
R_{f} (EE/MeOH 5:1) = 0,40 MS (DCl): 403 (M + 1)

### b) 4-(Imidazol-2-yl)phenoxy-3-methylsulfonyl-benzoylguanidin, Dihydrochlorid

0,5 mmol Ester 2a) und 2,5 mmol Guanidin werden in 3 ml Isopropanol nach Variante B umgesetzt. 100 mg, mp. 210°C (Zers.)
R_{f} (EE/MeOH 5:1) = 0,06 MS (ES): 400 (M + 1)

### Beispiel 3

### 4-(3-Pyridylphenoxy)-3-methylsulfonyl-benzoylguanidin, Dihydrochlorid

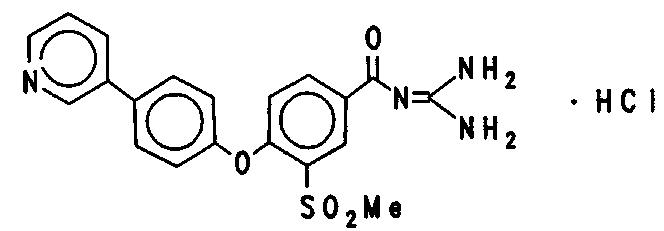

### a) 4-(3-Pyridyl)-anisol

5 mmol 3-Brompyridin werden in 30 ml Toluol und 8 ml Ethanol gelöst und 0,25 mmol (CH₃COO)₂ Pd, 0,5 mmol Triphenylphosphin, 5 ml einer 2N wäßrigen Na₂CO₃-Lösung sowie 5,5 mmol 4-Methoxyphenylboronsäure zugegeben. 30 Minuten lang wird unter Rückfluß gekocht, anschließend abgekühlt und auf 100 ml NaHCO₃-Lösung gegossen. Dann wird 3x mit je 100 ml EE extrahiert, über Na₂SO₄ getrocknet, das Solvens im Vakuum entfernt und an Kieselgel mit MTB chromatographiert. Man erhält 720 mg eines farblosen Öls.
R_{f} (EE) = 0,46 MS (DCl): 186 (M + 1)

### b) 4-(3-Pyridyl)-phenol

3,7 mmol Anisol 3a) werden in 10 ml Eisessig und 10 ml 48 % wäßriger HBr-Lösung 3 Stunden zum Rückfluß erhitzt. Nach dem Abkühlen wird auf 150 ml Na₂CO₃-Lösung gegossen und 3x mit je 150 ml EE extrahiert. Über Na₂SO₄ wird getrocknet und das Solvens im Vakuum entfernt. Man erhält 580 mg blaßgelber Kristalle, die ohne weitere Reinigung verwendet werden. mp. 197°C
R_{f} (MTB) = 0,34 MS (DCl): 172 (M + 1)

### c) 4-[4-(3-Pyridyl)phenoxy]-3-methylsulfonyl-benzoesäure-methylester

3,1 mmol 4-Brom-3-methylsulfonyl-benzoesäure-methylester, 3,1 mmol Phenol 3b) sowie 9,3 mmol K₂CO₃ werden in 30 ml DMF (wasserfrei) 3 Stunden bei 130°C gerührt. Nach dem Abkühlen wird auf 70 ml Na₂CO₃-Lösung gegossen und 3x mit je 100 ml EE extrahiert. Über Na₂SO₄ wird getrocknet, das Solvens im Vakuum entfernt und an Kieselgel mit EE chromatographiert. Man erhält 890 mg weißer Kristalle, mp. 145 bis 146°C.
R_{f} (EE) = 0,40 MS (DCl): 384 (M + 1)

### d) 4-[4-(3-Pyridyl)phenoxy]-3-methylsulfonyl-benzoylguanidn, Dihydrochlorid

2,2 mmol Ester 3c) werden nach Variante B in das Acylguanidin überführt.
190 mg weiße Kristalle mp. 270°C.
R_{f} (EE/MeOH 10:1) = 0,14 MS (ES): 411 (M + 1)

Die Titelverbindungen der Beispiele 4 bis 6 werden analog Beispiel 3 synthetisiert:

### Beispiel 4

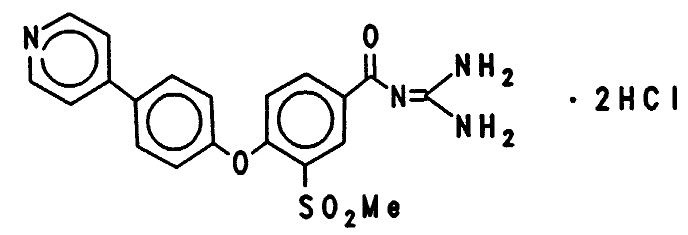

### 4-[4-(4-Pyridyl)phenoxy]-3-methylsulfonyl-benzoylguanidin, Dihydrochlorid

mp. 178 bis 179°C
R_{f} (EE/MeOH 5:1) = 0,29 MS (ES): 411 (M + 1)

### Beispiel 5

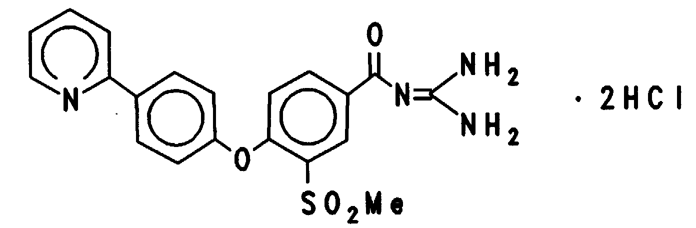

### 4-[4-(2-Pyridyl)phenoxy]-3-methylsulfonyl-benzoylguanidin, Dihydrochlorid

mp. 220 bis 223°C
R_{f} (EE/MeOH 5:1) = 0,44 MS (ES): 411 (M + 1)

### Beispiel 6

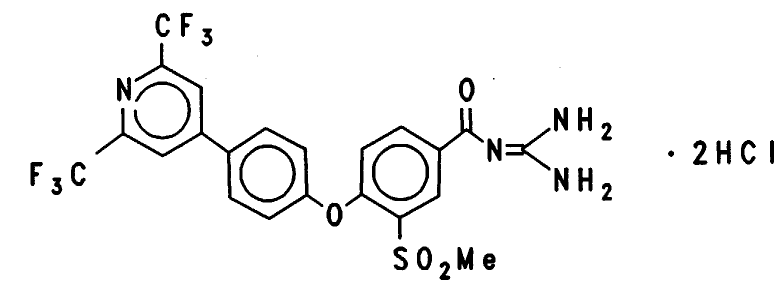

### 4-[4-(2,6-Bistrifluormethylpyridin-4-yl)-phenoxy]-3-methylsulfonyl-benzoylguanidin, Dihydrochlorid

mp. 267°C
R_{f} (EE) = 0,26 MS (ES): 547 (M + 1)

### a) 2,6-Bistrifluormethyl-4-brom-pyridin

4 mmol 2,6-Bistrifluormethyl-4-chlor-pyridin werden in 20 ml 33 % HBr in Eisessig gelöst und 10 Stunden bei 100°C gerührt. Man gießt auf 100 ml Na₂CO₃-Lösung, und es wird 3x mit je 100 ml Diethylether extrahiert. Das Solvens wird unter Normaldruck vorsichtig entfernt, und man erhält 940 mg einer farblosen Flüssigkeit, die ohne weitere Reinigung wie unter 3a) beschrieben verwendet wird.

Beispiel 7 wurde analog Beispiel 2 synthetisiert:

### Beispiel 7

### 3-(Imidazol-2-yl)phenoxy-3-methylsulfonyl-benzoylguanidin, Dihydrochlorid

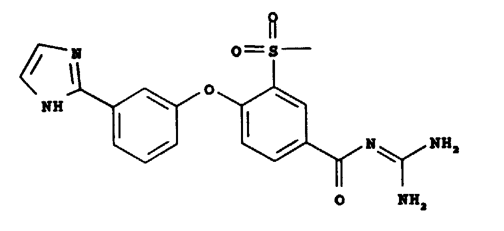

mp. >200°C (Zersetzung)
R_{f} (EE/MeOH 3:1) = 0.18 MS (ES) : 400 (M + 1)

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2 % Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Mediums (mmol/l: 140 NaCl, 3 KCl, 150 Sucrose, 0,1 Ouabain, 20 Tris-hydroxymethyl-aminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-lnflux ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

Inhibition des Na⁺/H⁺-Exchangers:

| Beispiel | IC₅₀ mol/l |
|---|---|
| 1 | 3,0 x 10⁻⁷ |
| 2 | 3,0 x 10⁻⁷ |
| 3 | 2,0 x 10⁻⁷ |
| 5 | 3,0 x 10⁻⁷ |

## Patentansprüche

1. Benzoylguanidin der Formel I 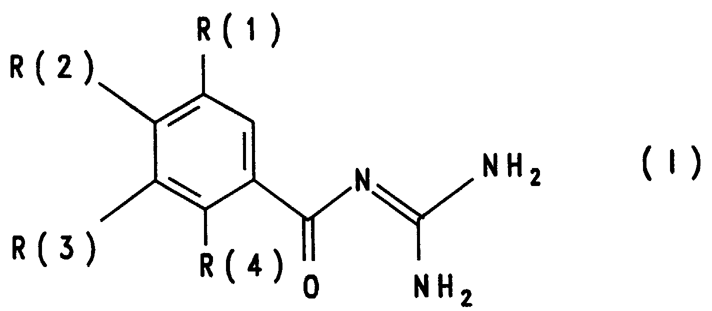 worin bedeuten:
R(1) Wasserstoff, F, Cl, Br, l, -NO₂, -C≡N, -Xₒ-(CH₂)ₚ-(CF₂)_{q}-CF₃, R(5)-SOₘ-, R(6)-CO-, R(6)R(7)N-CO- oder R(6)R(7)N-SO₂-;
X Sauerstoff, -S- oder NR(14);
m Null, 1 oder 2;
o Null oder 1;
p Null, 1 oder 2;
q Null, 1, 2, 3, 4, 5 oder 6;
R(5) und R(6) (C₁-C₈)-Alkyl, (C₃-C₆)-Alkenyl, -CₙH₂ₙ-R(8) oder CF₃;
n Null, 1, 2, 3 oder 4;
R(8) (C₃-C₇)-Cycloalkyl oder Phenyl, welches nicht substituiert ist oder substituiert mit 1 bis 3 Substituenten ausgewählt aus der Gruppe bestehend aus F, Cl, CF₃, Methyl, Methoxy und NR(9)R(10);
R(9) und R(10)
H oder (C₁-C₄)-Alkyl;
oder
R(6) Wasserstoff;
R(7) H oder (C₁-C₄)-Alkyl;
oder
R(6) und R(7) gemeinsam 4 oder 5 Methylengruppen, von denen eine CH₂-Gruppe durch Sauerstoff, S, NH, N-CH₃ oder N-Benzyl ersetzt sein kann;
R(2) 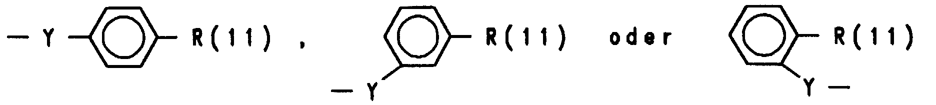
R(11) Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
die über C oder N verknüpft sind und die unsubstituiert oder substituiert sind mit 1 bis 3 Substituenten ausgewählt aus der Gruppe bestehend aus F, Cl, CF₃, CH₃, Methoxy, Hydroxy, Amino, Methylamino, Dimethylamino und Benzyl;
Y Sauerstoff, -S- oder NR(12)-;
R(12) H oder (C₁-C₄)-Alkyl;
R(3) wie R(1) definiert,
oder
R(3) (C₁-C₆)-Alkyl oder -X-R(13);
X Sauerstoff, -S- oder NR(14);
R(14)
Wasserstoff oder (C₁-C₃)-Alkyl;
R(13) H, (C₁-C₆)-Alkyl, (C₃-C₈)-Cycloalkyl oder -C_{b}H_{2b}-R(15);
b Null, 1, 2, 3 oder 4;
oder
R(13) und R(14) gemeinsam 4 oder 5 Methylengruppen, von denen eine CH₂-Gruppe durch Sauerstoff, S, NH, N-CH₃ oder N-Benzyl ersetzt sein kann,
R(15) Phenyl, das unsubstituiert oder substituiert ist mit 1 - 3 Substituenten ausgewählt aus der Gruppe bestehend aus F, Cl, CF₃, Methyl, Methoxy und NR(9)R(10);
R(9) und R(10)
H oder (C₁-C₄)-Alkyl;
R(4) Wasserstoff, -OR(16), -NR(16)R(17) oder CᵣF₂ᵣ₊₁;
R(16) und R(17) unabhängig voneinander Wasserstoff oder (C₁-C₃)-Alkyl;
r 1, 2, 3 oder 4;
sowie deren pharmazeutisch verträgliche Salze.

2. Benzoylguanidin der Formel I nach Anspruch 1, in der bedeuten:
R(1) Wasserstoff, F, Cl, -C=N, -CF₃, R(5)-SOₘ-, R(6)-CO-, R(6)R(7)N-CO- oder R(6)R(7)N-SO₂-;
m wie in Anspruch 1 definiert;
R(5) und R(6) (C₁-C₈)-Alkyl, (C₃-C₄)-Alkenyl, -CₙH₂ₙ-R(8) oder -CF₃;
n Null oder 1;
R(8) (C₃-C₆)-Cycloalkyl oder Phenyl, welches nicht substituiert ist oder substituiert wie in Anspruch 1 definiert;
wobei R(6) auch in der Bedeutung von H steht,
R(7) H oder Methyl,
R(3) Wasserstoff, Methyl, Cyano, -CF₃, F, Cl;
und die übrigen Reste wie in Anspruch 1 definiert sind.

3. Benzoylguanidin der Formel I nach Anspruch 1, in der bedeuten:
R(1) wie in Anspruch 2 definiert,
R(5) Methyl oder CF₃;
R(6) und R(7) unabhängig voneinander Wasserstoff oder Methyl;
R(2) wie in Anspruch 1 definiert;
R(12) Wasserstoff oder Methyl;
R(3) wie in Anspruch 2 definiert;
R(4) Wasserstoff, OH, NH₂ oder CF₃.

4. Benzoylguanidin der Formel I nach Anspruch 1, in der bedeuten:
R(1) wie in Anspruch 2 definiert;
R(5), R(6) und R(7) wie in Anspruch 3 definiert;
R(2) wie in Anspruch 1 definiert
R(11) Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl oder Pyridazinyl;
die über C oder N verknüpft sind und die unsubstituiert oder substituiert sind mit 1 bis 2 Resten ausgewählt aus der Gruppe bestehend aus F, Cl, CF₃, CH₃, Methoxy, Dimethylamino und Benzyl;
Y wie in Anspruch 1 definiert;
R(12) wie in Anspruch 3 definiert;
R(3) wie in Anspruch 2 definiert;
R(4) wie in Anspruch 3 definiert.

5. Benzoylguanidin der Formel I nach Anspruch 1, in der bedeuten:
R(1) wie in Anspruch 2 definiert;
R(5), R(6) und R(7 wie in Anspruch 3 definiert;
R(2) wie in Anspruch 1 definiert,
R(11) Pyrrolyl, Imidazolyl, Pyrazolyl oder Pyridyl,
die unsubstituiert oder substituiert sind mit 1 bis 2 Resten ausgewählt aus der Gruppe bestehend aus F, Cl, CF₃, CH₃, Methoxy, Dimethylamino und Benzyl;
Y wie in Anspruch 1 definiert;
R(12) wie in Anspruch 3 definiert.
R(3) wie in Anspruch 2 definiert;
R(4) wie in Anspruch 3 definiert.

6. Verfahren zur Herstellung eines Benzoylguanidins der Formel I nach Anspruch 1, dadurch gekennzeichnet, daß man eine Verbindung der Formel II 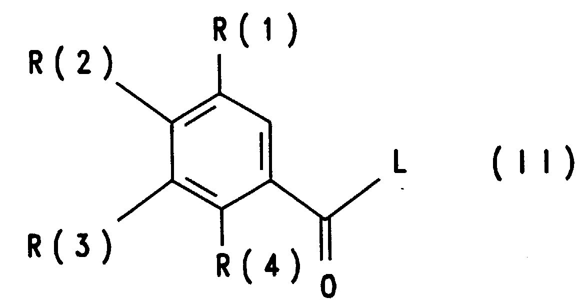 mit Guanidin umsetzt, worin R(1) bis R(4) die angegebene Bedeutung besitzen und L für eine leicht nucleophil substituierbare leaving group steht.

7. Verwendung einer Verbindung I nach Anspruch 1 zur Herstellung eines Medikaments zu Behandlung von Arrhythmien.

8. Verwendung einer Verbindung I nach Anspruch 1 zur Herstellung eines Medikaments zur Behandlung oder Prophylaxe des Herzinfarktes.

9. Verwendung einer Verbindung I nach Anspruch 1 zur Herstellung eines Medikaments zur Behandlung oder Prophylaxe der Angina Pectoris.

10. Verwendung einer Verbindung I nach Anspruch 1 zur Herstellung eines Medikaments zur Behandlung oder Prophylaxe von ischämischen Zuständen des Herzens.

11. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

12. Verwendung einer Verbindung I nach Anspruch 1 zur Herstellung eines Medikaments zur Behandlung oder Prophylaxe von ischämischen Zuständen peripherer Organe und Gliedmaßen.

13. Verwendung einer Verbindung I nach Anspruch 1 zur Herstellung eines Medikaments zur Behandlung von Schockzuständen.

14. Verwendung einer Verbindung I nach Anspruch 1 zur Herstellung eines Medikaments zum Einsatz bei chirurgischen Operationen und Organtransplantationen.

15. Verwendung einer Verbindung I nach Anspruch 1 zur Herstellung eines Medikaments zur Konservierung und Lagerung von Transplantaten für chirurgische Maßnahmen.

16. Verwendung einer Verbindung I nach Anspruch 1 zur Herstellung eines Medikaments zur Behandlung von Krankheiten, bei denen die Zellproliferation eine primäre oder sekundäre Ursache darstellt, und somit ihre Verwendung als Antiatherosklerotika, Mittel gegen Diabetische Spätkomplikationen, Krebserkrankungen, fibrotische Erkrankungen wie Lungenfibrose, Leberfibrose oder Nierenfibrose, Prostatahyperplasie.

17. Verwendung einer Verbindung I nach Anspruch 1 zur Herstellung eines wissenschaftliches Tools zur Inhibition des Na⁺/H⁺-Exchangers, zur Diagnose der Hypertonie und proliferativer Erkrankungen.

18. Heilmittel, gekennzeichnet durch einen wirksamen Gehalt einer Verbindung der Formel I nach Anspruch 1.

## Claims

1. A benzoylguanidine of the formula I 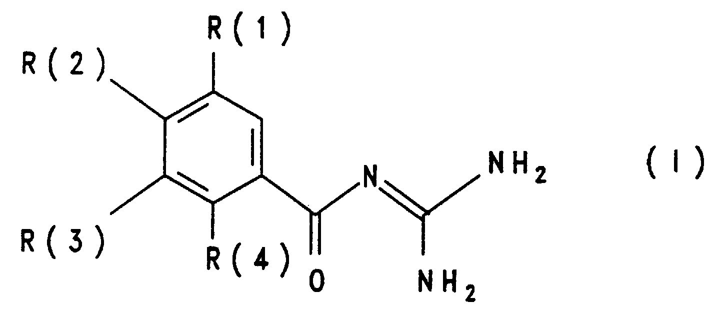 in which :
R(1) is hydrogen, F, Cl, Br, I, -NO₂, -C≡N, -Xₒ-(CH₂)ₚ- (CF₂)_{q}-CF₃, R(5)-SOₘ-, R(6)-CO-, R(6)R(7)N-CO- or R(6)R(7)N-SO₂-;
X is oxygen, -S- or NR(14);
m is zero, 1 or 2;
o is zero or 1;
p is zero, 1 or 2;
q is zero, 1, 2, 3, 4, 5 or 6;
R(5) and R(6) are (C₁-C₈)-alkyl, (C₃-C₆)-alkenyl, -CₙH₂ₙ-R(8) or CF₃;
n is zero, 1, 2, 3 or 4;
R(8) is (C₃-C₇)-cycloalkyl, or phenyl which is unsubstituted or substituted by 1 to 3 substituents selected from the group consisting of F, Cl, CF₃, methyl, methoxy and NR(9)R(10);
R(9) and R(10) are
H or (C₁-C₄)-alkyl;
or
R(6) is hydrogen;
R(7) is H or (C₁-C₄)-alkyl;
or
R(6) and R(7) together are 4 or 5 methylene groups, of which one CH₂ group can be replaced by oxygen, S, NH, N-CH₃ or N-benzyl;
R(2) is 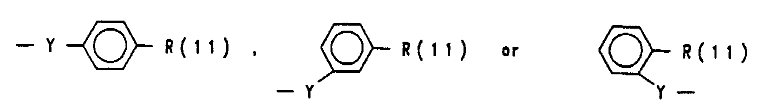
R(11) is furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl, which are linked via C or N and which are unsubstituted or substituted by 1 to 3 substituents selected from the group consisting of F, Cl, CF₃, CH₃, methoxy, hydroxyl, amino, methylamino, dimethylamino and benzyl;
Y is oxygen, -S- or NR(12)-;
R(12) is H or (C₁-C₄)-alkyl;
R(3) is defined as R(1),
or
R(3) is (C₁-C₆)-alkyl or -X-R(13);
X is oxygen, -S- or NR(14);
R(14) is hydrogen or (C₁-C₄)-alkyl;
R(13) is H, (C₁-C₆)-alkyl, (C₃-C₈)-cycloalkyl or -C_{b}H_{2b}-R(15) ;
b is zero, 1, 2, 3 or 4;
or
R(13) and R(14) together are 4 or 5 methylene groups, of which one CH₂ group can be replaced by oxygen, S, NH, N-CH₃ or N-benzyl,
R(15) is
phenyl which is unsubstituted or substituted by 1 - 3 substituents selected from the group consisting of F, Cl, CF₃, methyl, methoxy and NR(9)R(10);
R(9) and R(10) are
H or (C₁-C₄)-alkyl;
R(4) is hydrogen, -OR(16), -NR(16)R(17) or CᵣF₂ᵣ₊₁ ;
R(16) and R(17) independently of one another are hydrogen or (C₁-C₃)-alkyl;
r is 1, 2, 3 or 4;
and pharmaceutically tolerated salts thereof.

2. A benzoylguanidine of the formula I as claimed in claim 1, in which:
R(1) is hydrogen, F, Cl, -C≡N, -CF₃, R(5)-SOₘ-, R(6)-CO-, R(6)R(7)N-CO- or R(6)R(7)N-SO₂-;
m is defined as in claim 1;
R(5) and R(6) are
(C₁-C₈)-alkyl, (C₃-C₄)-alkenyl, -CₙH₂ₙ-R(8) or -CF₃;
n is zero or 1;
R(8) is (C₃-C₆)-cycloalkyl or phenyl, which is unsubstituted or substituted as defined in claim 1;
where R(6) alternatively has the meaning of H,
R(7) is H or methyl,
R(3) is hydrogen, methyl, cyano, -CF₃, F or Cl; and the other radicals are defined as in claim 1.

3. A benzoylguanidine of the formula I as claimed in claim 1, in which:
R(1) is defined as in claim 2,
R(5) is methyl or CF₃;
R(6) and R(7) independently of one another are hydrogen or methyl;
R(2) is defined as in claim 1;
R(12) is hydrogen or methyl;
R(3) is defined as in claim 2;
R(4) is hydrogen, OH, NH₂ or CF₃.

4. A benzoylguanidine of the formula I as claimed in claim 1, in which:
R(1) is defined as in claim 2;
R(5), R(6) and R(7) are defined as in claim 3;
R(2) is defined as in claim 1
R(11) is furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl or pyridazinyl; which are linked via C or N and which are unsubstituted or substituted by 1 to 2 radicals selected from the group consisting of F, Cl, CF₃, CH₃, methoxy, dimethylamino and benzyl;
Y is defined as in claim 1;
R(12) is defined as in claim 3;
R(3) is defined as in claim 2;
R(4) is defined as in claim 3.

5. A benzoylguanidine of the formula I as claimed in claim 1, in which:
R(1) is defined as in claim 2;
R(5), R(6) and R(7) are defined as in claim 3;
R(2) is defined as in claim 1,
R(11) is pyrrolyl, imidazolyl, pyrazolyl or pyridyl,
which are unsubstituted or substituted by 1 to 2 radicals selected from the group consisting of F, Cl, CF₃, CH₃, methoxy, dimethylamino and benzyl;
Y is defined as in claim 1;
R(12) is defined as in claim 3;
R(3) is defined as in claim 2;
R(4) is defined as in claim 3.

6. A process for preparing a benzoylguanidine of the formula I as claimed in claim 1, which comprises reacting a compound of the formula II 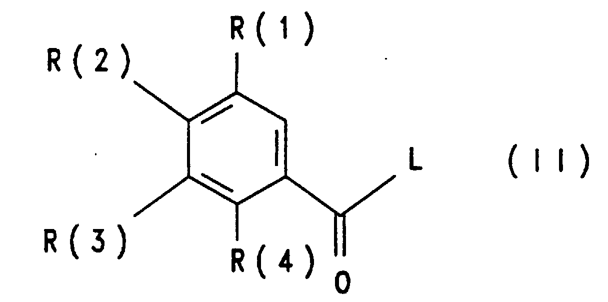 in which R(1) to R(4) have the meaning given and L is a leaving group which can readily be substituted nucleophilically, with guanidine.

7. Use of a compound I as claimed in claim 1 for preparing a medicament for treating arrhythmias.

8. Use of a compound I as claimed in claim 1 for preparing a medicament for the treatment or prophylaxis of cardiac infarction.

9. Use of a compound I as claimed in claim 1 for preparing a medicament for the treatment or prophylaxis of angina pectoris.

10. Use of a compound I as claimed in claim 1 for preparing a medicament for the treatment or prophylaxis of ischemic conditions of the heart.

11. Use of a compound I as claimed in claim 1 for preparing a medicament for the treatment or prophylaxis of ischemic conditions of the peripheral and central nervous system and of stroke.

12. Use of a compound I as claimed in claim 1 for preparing a medicament for the treatment or prophylaxis of ischemic conditions of peripheral organs and limbs.

13. Use of a compound I as claimed in claim 1 for preparing a medicament for the treatment of conditions of shock.

14. Use of a compound I as claimed in claim 1 for preparing a medicament for employment in surgical operations and organ transplantations.

15. Use of a compound I as claimed in claim 1 for preparing a medicament for the preservation and storage of transplants for surgical measures.

16. Use of a compound I as claimed in claim 1 for preparing a medicament for the treatment of illnesses in which cell proliferation represents a primary or secondary cause, and thus their use as antiatherosclerotic agents, and agents against diabetic secondary complications, cancers, fibrotic disorders, such as pulmonary fibrosis, hepatic fibrosis or renal fibrosis, and prostate hyperplasia.

17. Use of a compound I as claimed in claim 1 for preparing a scientific tool for inhibiting the Na⁺/H⁺ exchanger, and for diagnosing hypertension and proliferative disorders.

18. A medicine, comprising an effective amount of a compound of the formula I as claimed in claim 1.

## Revendications

1. Benzoylguanidine de formule I 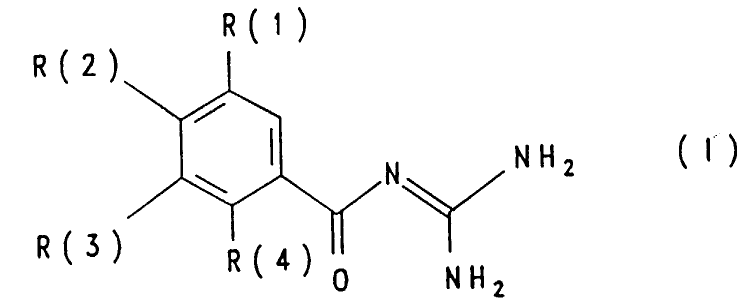 dans laquelle
R(1) représente un atome d'hydrogène ou de F, Cl, Br, I, ou un groupe -NO₂, -C≡N, -Xₒ-(CH₂)ₚ-(CF₂)_{q}-CF₃, R(5)-SOₘ, R(6)-CO-, R(6)R(7)N-CO- ou R(6)R(7)N-SO₂-,
X représente un atome d'oxygène, -S- ou NR(14),
m est zéro, 1 ou 2,
o est zéro ou 1,
p est zéro, 1 ou 2,
q est zéro, 1, 2, 3, 4, 5 ou 6,
R(5) et R(6) représentent
un groupe alkyle en C₁-C₈, alcényle en C₃-C₆, CₙH₂ₙ-R(8) ou CF₃,
n est zéro, 1, 2, 3 ou 4,
R(8) représente un groupe cycloalkyle en C₃-C₇, ou phényle
qui est non substitué ou substitué par 1-3 substituants choisis parmi F, Cl, CF₃, le groupe méthyle, méthoxy ou NR(9)R(10),
R(9) et R(10) représentant
H ou un groupe alkyle en C₁-C₄,
ou
R(6) représente un atome d'hydrogène,
R(7) représente un atome d'hydrogène ou un groupe alkyle en C₁-C₄, ou
R(6) et R(7) peuvent être ensemble 4 ou 5 groupes méthylène, dont un groupe CH₂ peut être remplacé par un atome d'oxygène ou de S ou par un groupe NH, N-CH₃ ou N-benzyle,
R(2) représente 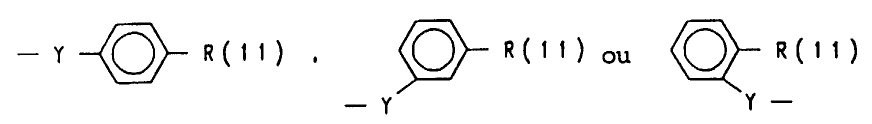
R(11) représente un groupe furannyle, thiényle, pyrrolyle, imidazolyle, pyrazolyle, triazolyle, tétrazolyle, oxazolyle, isoxazolyle, thiazolyle, isothiazolyle, pyridyle, pyrazinyle, pyrimidinyle, pyridazinyle, indolyle, indazolyle, quinolyle, isoquinolyle, phtalazinyle, quinoxalinyle, quinazolinyle ou cinnolinyle,
qui sont liés par C ou N et qui sont non substitués ou substitués par 1-3 substituants choisis dans le groupe constitué par F, Cl, les groupes CF₃, CH₃, méthoxy, hydroxy, amino, méthylamino, diméthylamino et benzyle,
Y représente un atome d'oxygène, -S- ou NR(12), R(12) représentant H ou un groupe alkyle en C₁-C₄,
R(3) est défini comme R(1),
ou
R(3) représente un groupe alkyle en C₁-C₆ ou -X-R(13),
X représente un atome d'oxygène, -S- ou NR(14), R(14) représente
H ou un groupe alkyle en C₁-C₃,
R(13) représente
H ou un groupe alkyle en C₁-C₆, cycloalkyle en C₃-C₈ ou C_{b}H_{2b}-R(15),
b étant zéro, 1, 2, 3 ou 4,
ou
R(13) et R(14) peuvent également représenter ensemble 4 ou 5 groupes méthylène, dont un groupe CH₂ peut être remplacé par un atome d'oxygène ou de S ou par un groupe NH, N-CH₃ ou N-benzyle,
R(15) représente un groupe phényle qui est non substitué ou substitué par 1-3 substituants choisis parmi F, Cl, CF₃, méthyle, méthoxy et NR(9)R(10),
R(9) et R(10)
représentant H ou un groupe alkyle en C₁-C₄,
R(4) représente un atome d'hydrogène ou un groupe -OR(16), -NR(16)R(17) ou CᵣF₂ᵣ₊₁,
R(16) et R(17) représentant, indépendamment l'un de l'autre, un atome d'hydrogène ou un groupe alkyle en C₁-C₃,
r étant 1, 2, 3 ou 4,
et ses sels pharmaceutiquement acceptables.

2. Benzoylguanidine de formule I selon la revendication 1, dans laquelle
R(1) représente un atome d'hydrogène ou de F, Cl, -C≡N, -CF₃, R(5)-SOₘ-, R(6)-CO-, R(6)R(7)N-CO-, ou R(6)R(7)N-SO₂-,
m est tel que défini dans la revendication 1,
R(5) et R(6) représentent un groupe alkyle en C₁-C₈, alcényle en C₃-C₄, -CₙH₂ₙ-R(8) ou CF₃,
n est zéro ou 1,
R(8) représente un groupe cycloalkyle en C₃-C₆ ou phényle qui est substitué comme dans la revendication 1,
R(6) représentant également H,
R(7) représente H ou le groupe méthyle,
R(3) représente un atome d'hydrogène ou de F, CI ou le groupe méthyle, cyano ou -CF₃,
et les autres radicaux sont tels que définis dans la revendication 1.

3. Benzoylguanidine de formule I selon la revendication 1, dans laquelle
R(1) est tel que défini dans la revendication 2,
R(5) représente le groupe méthyle ou CF₃,
R(6) et R(7) représentent, indépendamment l'un de l'autre, un atome d'hydrogène ou le groupe méthyle,
R(2) est tel que défini dans la revendication 1,
R(12) représente un atome d'hydrogène ou le groupe méthyle,
R(3) est tel que défini dans la revendication 2,
R(4) représente un atome d'hydrogène ou un groupe OH, NH₂ ou CF₃.

4. Benzoylguanidine de formule I selon la revendication 1, dans laquelle
R(1) est tel que défini dans la revendication 2,
R(5), R(6) et R(7) sont tels que définis dans la revendication 3,
R(2) est tel que défini dans la revendication 1
R(11) représente un groupe furannyle, thiényle, pyrrolyle, imidazolyle, pyrazolyle, triazolyle, tétrazolyle, oxazolyle, isoxazolyle, thiazolyle, isothiazolyle, pyridyle, pyrazinyle, pyrimidinyle, pyridazinyle,
qui sont liés par C ou N et qui sont non substitués ou substitué par 1 ou 2 radicaux choisis dans le groupe constitué par F, CI et les groupes CF₃, CH₃, méthoxy, diméthylamino et benzyle,
Y est tel que défini dans la revendication 1, R(12) est tel que défini dans la revendication 3,
R(3) est tel que défini dans la revendication 2,
R(4) est tel que défini dans la revendication 3.

5. Benzoylguanidine de formule I selon la revendication 1, dans laquelle
R(1) est tel que défini dans la revendication 2,
R(5), R(6) et R(7) sont tels que définis dans la revendication 3,
R(2) est tel que défini dans la revendication 1
R(11) représente un groupe pyrrolyle, imidazolyle, pyrazolyle ou pyridyle,
qui sont non substitués ou substitué par 1 ou 2 radicaux choisis dans le groupe constitué par F, CI et les groupes CF₃, CH₃, méthoxy, diméthylamino et benzyle,
Y est tel que défini dans la revendication 1, R(12) est tel que défini dans la revendication 3,
R(3) est tel que défini dans la revendication 2,
R(4) est tel que défini dans la revendication 3.

6. Procédé pour la préparation d'une benzoylguanidine de formule I selon la revendication 1, caractérisé en ce que l'on fait réagir un composé de formule II 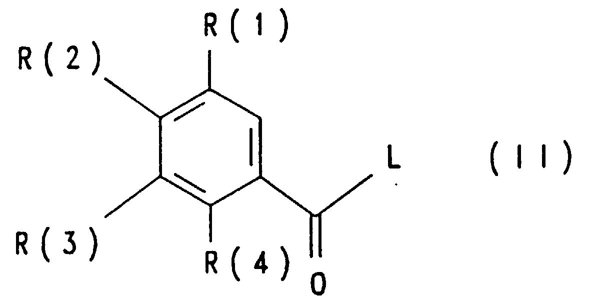 avec de la guanidine, formule dans laquelle R(1) à R(4) ont les significations données et L représente un groupe partant pouvant être aisément remplacé par substitution nucléophile.

7. Utilisation d'un composé I selon la revendication 1, pour la fabrication d'un médicament destiné au traitement d'arythmies.

8. Utilisation d'un composé I selon la revendication 1, pour la fabrication d'un médicament destiné au traitement ou à la prophylaxie de l'infarctus du myocarde.

9. Utilisation d'un composé I selon la revendication 1, pour la fabrication d'un médicament destiné au traitement ou à la prophylaxie de l'angine de poitrine.

10. Utilisation d'un composé I selon la revendication 1, pour la fabrication d'un médicament destiné au traitement ou à la prophylaxie d'états ischémiques du coeur.

11. Utilisation d'un composé I selon la revendication 1, pour la fabrication d'un médicament destiné au traitement ou à la prophylaxie d'états ischémiques du système nerveux périphérique et central et de l'apoplexie cérébrale.

12. Utilisation d'un composé I selon la revendication 1, pour la fabrication d'un médicament destiné au traitement ou à la prophylaxie d'états ischémiques d'organes périphériques et des membres.

13. Utilisation d'un composé I selon la revendication 1, pour la fabrication d'un médicament destiné au traitement d'états de choc.

14. Utilisation d'un composé I selon la revendication 1, pour la fabrication d'un médicament destiné à l'utilisation dans des opérations chirurgicales et des greffes d'organes.

15. Utilisation d'un composé I selon la revendication 1, pour la fabrication d'un médicament destiné à la conservation et au stockage de transplants pour des opérations chirurgicales.

16. Utilisation d'un composé I selon la revendication 1, pour la fabrication d'un médicament destiné au traitement de maladies dans lesquelles la prolifération cellulaire représente une cause primaire ou secondaire, et par conséquent son utilisation en tant qu'agent anti-athérosclérose, agent contre des complications tardives diabétiques, des maladies cancéreuses, des fibroses telles que la fibrose pulmonaire, la fibrose hépatique ou la fibrose rénale, l'hyperplasie de la prostate.

17. Utilisation d'un composé I selon la revendication 1, pour la fabrication d'un outil scientifique pour l'inhibition de l'échangeur Na⁺/H⁺, pour le diagnostic de l'hypertonie et de maladies proliférantes.

18. Médicament caractérisé par une teneur efficace en un composé de formule I selon la revendication 1.
